# FASCICULE DE BREVET EUROPEEN

(11) **EP 1 849 111 B1**
(45) Date de publication et mention de la délivrance du brevet: **26.08.2020**
(21) Numéro de dépôt: 06709236.1
(22) Date de dépôt: 03.02.2006
(51) Int. Cl.: A61F 13/08

(54) **DISPOSITIF D'AIDE A LA SELECTION D'UNE ORTHESE DE CONTENTION PAR SIMULATION DE SES EFFETS SUR L'HEMODYNAMIQUE DU RETOUR VEINEUX**
EINRICHTUNG ZUR HILFE BEI DER AUSWAHL EINER KOMPRESSIVEN ORTHOSE DURCH SIMULIEREN IHRER EFFEKTE AUF DIE HEMODYNAMIK DES VENÖSEN RÜCKFLUSSES
DEVICE FOR ASSISTING IN THE SELECTION OF A COMPRESSIVE ORTHOSIS BY SIMULATING ITS EFFECTS UPON THE HEMODYNAMICS OF THE VENOUS RETURN

(30) Priorité: 16.02.2005 FR 0501569
(43) Date de publication de la demande: 31.10.2007
(73) Titulaire: Laboratoires Innothera, 94110 Arcueil (FR)
(72) Inventeur: GOBET, Arnaud, F-75007 Paris (FR)
(74) Mandataire: Nony
(86) Numéro de dépôt international: PCT/FR2006/000244
(87) Numéro de publication internationale: WO 2006/087442

(56) Documents cités:
- FR-A- 2 852 421
- "PROGRAMME FINAL CAHIER DES RESUMES" SOCIETE FRANÇAISE DE MEDECINE VASCULAIRE, CONGRES ANNUEL DE VASCULIS 2004, 9-11 SEPTEMBRE 2004, LYON, FRANCE, [Online] 2004, XP002342919 Extrait de l'Internet: URL:http://www.angioweb.fr/SFMV.pdf> [extrait le 2005-08-29]
- CHU T-M ET AL: "Three-dimensional finite element stress analysis of the polypropylene, ankle-foot orthosis: static analysis" MEDICAL ENGINEERING & PHYSICS, BUTTERWORTH-HEINEMANN, GB, vol. 17, no. 5, juillet 1995 (1995-07), pages 372-379, XP002277428 ISSN: 1350-4533
- SYNGELLAKIS S ET AL: "ASSESSMENT OF THE NON-LINEAR BEHAVIOUR OF PLASTIC ANKLE FOOT ORTHOSES BY THE FINITE ELEMENT METHOD" PROCEEDINGS OF THE INSTITUTION OF MECHANICAL ENGINEERS. JOURNAL OF ENGINEERING IN MEDICINE. PART H, MECHANICAL ENGINEERING PUBLICATIONS LTD, LONDON, GB, vol. 214, no. 5, PART H, 2000, pages 527-539, XP001161162 ISSN: 0954-4119
- CROS F ET AL: "A digital model for the venous junctions." COMPUTER METHODS IN BIOMECHANICS AND BIOMEDICAL ENGINEERING. DEC 2002, vol. 5, no. 6, décembre 2002 (2002-12), pages 421-429, XP008051806 ISSN: 1025-5842

## Description

L'invention concerne l'utilisation d'un dispositif pour évaluer l'efficacité d'une contention donnée sur le retour veineux, de manière à définir des profils de pression optimisés, puis, à partir des profils optimaux ainsi trouvés, choisir une orthèse dans une gamme préexistante ou fabriquer sur mesure une orthèse d'après les caractéristiques morphologiques d'un patient, telle que définie dans les revendications.

La divulgation concerne plus précisément les orthèses compressives destinées aux membres inférieurs telles que bas, collants ou chaussettes.

L'effet recherché est une contention/compression à partir de la cheville à un degré thérapeutique, avec dégressivité ou non,.

Fullana JM et coll., *The Venous Return Simulator: an Effective Tool for Investigating the Effects of External Compression on the Venous Hemo-⁻ dynamics* - *First Results after Thigh Compression,* VASA,2005;34 décrivent un outil mathématique de simulation du retour veineux, qui modélise le réseau veineux de la partie inférieure de la jambe, décomposé en une pluralité de segments représentatifs chacun d'une portion de vaisseau.

Ce modèle mathématique est basé sur des données physiologiques et anatomiques décrivant le réseau veineux du membre inférieur. Une fois définie la topographie du réseau à partir de ces données, le modèle évalue pour chaque segment le débit et la pression du sang qui y circule, en tenant compte de l'interaction entre les différents vaisseaux du fait de leur diamètre, de leur élasticité et de la manière dont ils sont interconnectés. La distribution des débits et des pressions dans le réseau est calculée avec des conditions aux limites comprenant : le débit sanguin en entrée, à partir de la région du pied et de diverses sources d'origine microcircula-toire ; des conditions de pression au point de sortie constitué par la veine fémorale commune ; ainsi qu'un paramètre de compression externe éventuellement exercée sur le membre.

L'article précité contient une comparaison des résultats fournis par le modèle mathématique avec des mesures effectuées par ailleurs sur une population de patients dans le cadre d'essais cliniques, pour des pressions de contention variables appliquées au moyen d'un manchon pneumatique placé au milieu de la cuisse. La comparaison montre une excellente concordance des résultats sur une large gamme de valeurs de pression appliquées par le manchon.

Ce modèle mathématique ne s'attache cependant pas à évaluer l'efficacité thérapeutique que l'on peut attendre dans des situations réelles avec des orthèses, telles que les bas ou collants couramment prescrits aux patients souffrant notamment d'insuffisance veineuse chronique.

Avec ces articles, la contention/compression recherchée est obtenue par le matériau élastique de l'orthèse, typiquement une maille tricotée de texture très serrée procurant l'effet thérapeutique recherché.

Pour ce faire, la maille et les fils de l'orthèse, ainsi que le dimensionnement des différentes rangées de maille, sont définis de manière à appliquer à différentes altitudes de la jambe différentes pressions prédéterminées, par exemple à la hauteur de la cheville, au départ du mollet, au niveau du mollet, au creux poplité, etc. jusqu'en haut de cuisse dans le cas d'un bas-cuisse ou d'un collant. Pour une altitude donnée, la structure de la rangée de mailles ainsi que le fil de trame exercent le long du contour du membre des forces de rappel élastique produisant localement la pression de contention voulue en surface du membre.

La pression est un facteur essentiel pour procurer un effet thérapeutique satisfaisant. Ainsi, un profil de pression inapproprié peut se traduire localement par certaines zones de contention excessive ou, inversement, insuffisante du membre (le "profil de pression" est la caractéristique décrivant la pression moyenne exercée par l'orthèse à une hauteur donnée du membre).

Les profils de pression sont actuellement évalués à partir de modèles de jambes en bois, par exemple les jambes dites "modèles Hohenstein", qui ne correspondent pas à la réalité morphologique, notamment du fait que ces modèles de jambes en bois présentent en section droite un contour circulaire conduisant à l'application, pour une hauteur donnée, d'une pression uniforme sur tout ce contour (selon la loi de Laplace, la pression exercée localement en un point du contour est inversement proportionnelle au rayon de courbure du contour en ce point de sorte que, pour une courbure constante, la pression est la même en tous points du contour). En revanche, sur un membre d'un patient, compte tenu des contours non circulaires la pression n'est plus exercée de manière aussi uniforme, de sorte que les modèles actuellement utilisés ne donnent qu'une image relativement lointaine de la compression effectivement exercée en tout point du membre - et donc, à plus forte raison, de l'effet thérapeutique réel sur le retour veineux qu'il est possible d'escompter à partir d'une telle compression.

Jusqu'à présent, l'efficacité réelle des orthèses ne pouvait être évaluée que dans le cadre d'essais cliniques, impliquant un examen du patient par un praticien équipé d'une instrumentation appropriée : pléthysmographe, matériel échographique, etc.

L'un des buts de l'invention est de proposer un dispositif permettant d'évaluer l'efficacité d'une contention donnée sur le retour veineux, de manière à définir des profils de pression optimisés. Cette invention permet ainsi de mieux cibler les investigations cliniques et en réduire le nombre.

À partir des profils optimaux ainsi trouvés, une orthèse particulière pourra être soit choisie dans une gamme préexistante, soit fabriquée sur mesure d'après les caractéristiques morphologiques du patient.

L'invention a également pour but de proposer un tel dispositif pouvant être utilisé comme outil de développement de nouvelles gammes d'orthèses, par simulation de l'action d'une contention donnée sur le retour veineux d'une population typique de patients.

Le concepteur d'orthèses pourra alors, à partir des résultats de cette simulation, rechercher les profils procurant le meilleur effet thérapeutique, avec des profils de pression différents et éventuellement plus complexes que les profils des gammes aujourd'hui disponibles.

Pour résoudre ces problèmes et pallier les limitations des techniques actuelles, l'invention propose essentiellement d'utiliser un modèle mathématique tel que celui décrit dans l'article précité de Fullana et coll. à des fins non plus théoriques, mais pour la définition de profils de pression permettant notamment : la sélection d'une orthèse appropriée dans une gamme préexistante ; la définition des paramètres de fabrication d'une orthèse sur mesure pour un patient donné ; ou encore la conception de nouveaux types d'orthèses présentant des profils de pression différents de ceux des produits disponibles actuellement sur le marché.

utilisé dans dispositif, tel Le dispositif l'inventlon est un que décrit dans la revendication 1, c'est à dire du type général divulgué par le document FR-A-2852421, et comprenant en outre moyens tels que ceux divulgués dans l'article précité de Fullana et coll., à savoir des moyens de simulation numérique de l'action de la contention sur l'hémodynamique du retour veineux, aptes à délivrer des valeurs de débit san-guin et/ou de pression intraveineuse en une pluralité de points d'un modèle numérique représentatif du réseau veineux jambier d'un membre, et des moyens de présentation desdites valeurs de débit sanguin et/ou de pression intraveineuse déterminées par ces moyens de simulation numérique.

De façon caractéristique de l'invention, ce dispositif comprend en outre des moyens pour : produire des valeurs de pression de contention susceptibles d'être exercées par l'orthèse en surface du membre en une pluralité correspondante de points prédéterminés, et appliquer les valeurs ainsi produites en entrée desdits moyens de simulation numérique. Les moyens pour produire les valeurs de pression de contention peuvent être des moyens de mesure, comprenant un ensemble de capteurs de mesure de la pression exercée par l'orthèse en surface du membre à l'endroit du capteur.

Ils peuvent également être des moyens de calcul, comprenant : des moyens pour établir un premier fichier de données représentatives des caractéristiques morphologiques du membre ; des moyens pour établir un deuxième fichier de données représentatives des caractéristiques dimensionnelles et rhéologiques de l'orthèse ; et des moyens de calcul de cartographie de pression de contention, aptes à déterminer lesdites valeurs de pression de contention à partir des données des premier et deuxième fichiers.

De tels moyens sont par exemple divulgués, en tant que tels, par le WO-A-2004/095342 (correspondant au FR-A-2 852 421), et également dans une communication au congrès De Vasculis de la Société française de médecine vasculaire, 9-11 sept. 2004, Lyon, France, pp. 10-11, mais il n'avait jamais été envisagé, jusqu'à la date de priorité de la présente demande, de combiner ces moyens avec un outil mathématique de simulation du retour veineux en appliquant, comme le propose l'invention, les données produites par les moyens de calcul en tant que paramètre d'entrée du modèle de réseau veineux, et ceci pour évaluer directement l'effet thérapeutique sur le retour veineux qu'il est possible d'escompter à partir d'une orthèse réelle.

Les moyens de simulation numérique reçoivent en entrée un paramètre de posture et sélectionner ou adapter le modèle numérique du réseau veineux en fonction de ce paramètre de posture.

De préférence, les moyens de présentation des valeurs de débit sanguin et/ou de pression intraveineuse-comprennent des moyens d'affichage d'un graphe orienté valué comprenant une pluralité d'arcs interconnectés représentatifs chacun d'un segment de vaisseau du réseau veineux jambier, les moyens d'affichage associant à chaque arc du graphe une valeur correspondante de débit sanguin et/ou de pression intraveineuse calculée par les moyens de simulation numérique, par exemple avec application locale à chaque arc du graphe d'un codage de couleur représentatif de la valeur correspondante de débit sanguin et/ou de pression intraveineuse. Les moyens d'affichage peuvent notamment produire une représentation plane du graphe et/ou, en superposition, une représentation tridimensionnelle du graphe et de la surface du membre.

On va maintenant décrire un exemple de mise en œuvre du dispositif utilisé dans l'invention, en référence aux dessins annexés.
La figure 1 illustre, de façon générale, les différents moyens mis en œuvre par le dispositif de l'invention pour permettre la sélection d'une orthèse de contention et/ou son adaptation à la morphologie du membre d'un patient.
La figure 2 montre la manière dont il est possible d'afficher sous forme graphique les résultats de la simulation numérique des valeurs de débit et de pression sanguine.

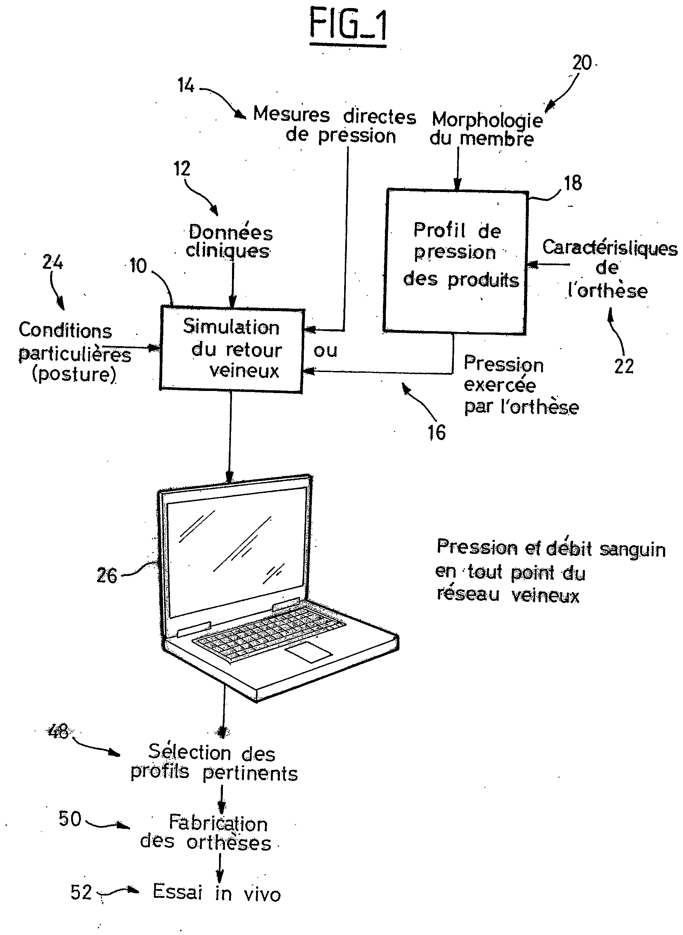

Sur la figure 1, la référence 10 désigne un bloc fonctionnel mettant en œuvre un logiciel de simulation du retour veineux, par exemple basé sur le modèle mathématique décrit dans l'article de Fullana et coll. cité plus haut.

Ce logiciel est paramétré à partir de données cliniques 12 permettant de définir une topologie du réseau veineux de la partie inférieure de la jambe, qui corresponde à un modèle typique représentatif d'une population de patients, éventuellement en fonction d'un tableau clinique correspondant à une pathologie particulière à laquelle on souhaite s'intéresser plus particulièrement (par exemple des veines jumelles dilatées).

Le logiciel de simulation du retour veineux reçoit en entrée des données de pression de contention (c'est-à-dire de la pression exercée en surface du membre) en une pluralité de points, notamment à plusieurs altitudes différentes de manière à définir un "profil de pression" correspondant.

Dans une première forme de mise en œuvre, ces pressions de contention proviennent de mesures directes 14 obtenues au moyen de dispositifs en eux-mêmes connus mettant en oeuvre des capteurs de pression. L'invention peut notamment être utilisée avec le dispositif décrit dans le WO-A-2004/000183 (Laboratoires Innothéra), qui décrit un dispositif en forme de manchon tubulaire permettant de produire un profil de contention prédéterminé sur un membre et mesurant simultanément la pression résultante appliquée. Un autre dispositif utilisable est celui décrit dans le WO-A-1998/058605, qui utilise un gabarit reproduisant une jambe de référence et équipé d'un réseau de capteurs permettant de mesurer les pressions de contention appliquées en une pluralité de points du gabarit par une orthèse enfilée dessus.

Dans une autre mise en oeuvre de l'invention, les données de pression de contention appliquées en entrée du logiciel de simulation 10 sont des données 16 issues d'un logiciel de calcul d'un profil de pression 18, tel que celui décrit dans le WO-A-2004/095342 précité (Laboratoires Innothéra). Il s'agit d'un logiciel utilisant, d'une part, les caractéristiques morphologiques 20 du membre à étudier et, d'autre part, les caractéristiques dimensionnelles et rhéologiques 22 de l'orthèse, pour calculer les valeurs de pression de contention susceptibles d'être exercées par l'orthèse sur le membre en une pluralité de points répartis selon un maillage tridimensionnel représentatif de la surface du membre.

Le logiciel de simulation du retour veineux 10, outre les valeurs de pression de contention mesurées 14 ou calculées 16, reçoit en entrée un paramètre correspondant à une condition particulière de posture du patient : position orthostatique, décubitus dorsal, marche, position assise, etc.

Le logiciel de simulation 10, à partir de ces différents paramètres d'entrée, produit des données représentatives de la pression intraveineuse et/ou du débit sanguin en tous points du réseau veineux jambier, c'est-à-dire sur chaque section de vaisseau de ce réseau.

Ces données peuvent être synthétisées et affichées sous forme graphique sur un écran 26.

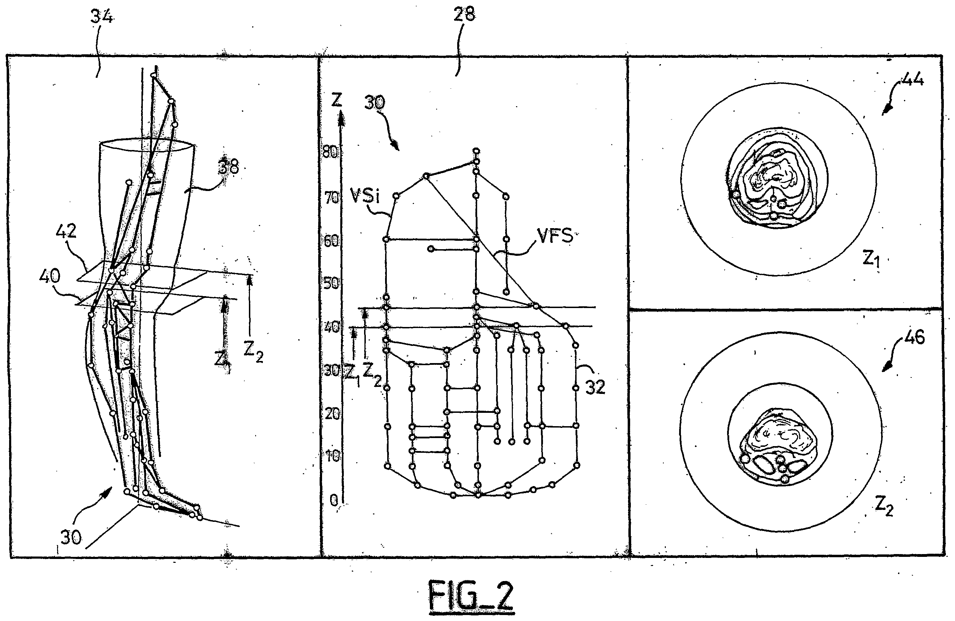

La figure 2 illustre un exemple d'affichage des données résultantes.

Une première fenêtre 28 donne une représentation plane du graphe modélisant le réseau veineux. Ce graphe 30 est un graphe orienté et valué formé d'une pluralité d'arcs 32 interconnectés représentatifs chacun d'un tronçon de vaisseau. Chaque arc est paramétré en fonction du diamètre et de l'élasticité du vaisseau. Le modèle tient compte du flux sanguin en entrée à partir de la région du pied et de diverses sources d'origine micro-circulatoire, et des conditions de pression en sortie dans la veine saphène interne VSI et la veine fémorale superficielle VFS.

Selon la contention appliquée en surface du membre, le praticien pourra évaluer visuellement et de façon synthétique l'effet en profondeur de cette contention, donc l'effet thérapeutique tel que prédit par le modèle.

Le débit et/ou la pression à l'intérieur de chaque vaisseau peuvent être représentés par une couleur différente. Si par exemple les débits sanguins les plus faibles sont affichés en bleu, et les plus élevés en rouge, par une modification des données d'entrée de pression de contention, l'opérateur verra immédiatement l'effet thérapeutique de cette modification de contention, non seulement de façon globale en sortie du réseau jambier (comme cela était le cas jusqu'à présent), mais également de façon spécifique, et en profondeur, sur telle ou telle section de vaisseau.

Dans une autre fenêtre 34 le logiciel de simulation affiche une représentation tridimensionnelle 36 du graphe plan 30 de la fenêtre 28. Cette représentation tridimensionnelle en perspective, avec possibilité de rotation sous contrôle de l'utilisateur, est avantageusement superposée à une représentation tridimensionnelle correspondante 38 de la surface du membre, pour faciliter la lecture. L'opérateur peut par ailleurs désigner des plans de coupe horizontaux tels que 40,42 à des altitudes respectives Z₁, Z₂ produisant dans des fenêtres respectives 44, 46 une représentation correspondante complète en coupe du membre avec os, vaisseaux, ligaments, etc.

À partir de ces investigations et observations, le praticien pourra alors par exemple définir (étape 48) quels sont les profils de pression les plus pertinents, c'est-à-dire ceux qui procurent l'effet thérapeutique optimal, et faire fabriquer à partir de ces profils des orthèses présentant les caractéristiques dimensionnelles et rhéologiques correspondantes (étape 50).

Cette sélection de profils pourra être enfin validée (étape 52) par des essais cliniques effectués *in vivo* sur des patients au moyen d'un appareillage approprié.

La mise en oeuvre de l'invention procure de nombreux avantages par rapport à la procédure antérieure, notamment :
- la possibilité d'étudier l'effet d'une contention sur des zones inaccessibles à la mesure, mais qui seront représentées sur le graphe affiché par le logiciel de simulation ;
- la vérification de la faisabilité de protocoles d'essais cliniques ;
- le ciblage des essais cliniques à réaliser effectivement, avec un gain de temps considérable dans la mesure où la sélection des profils pertinents est réalisée a *priori,* et non a *posteriori* ;
- un gain de temps considérable pour le développement de nouveaux produits mieux adaptés à leur cible, par exemple de produits destinés à traiter des pathologies spécifiques du réseau veineux jambier ;
- la possibilité de rechercher les meilleurs rapports dose/efficacité (c'est-à-dire l'effet obtenu en profondeur en fonction de la contention appliquée en surface), en évitant notamment d'exercer sur certaines zones des contentions inutiles, inconfortables pour le patient et même peut-être néfastes, pour concentrer la contention sur les régions où celle-ci, même modérée, procure une augmentation importante du débit veineux.

## Revendications

1. Utilisation d'un dispositif comprenant :
- des moyens pour produire des valeurs de pression de contention susceptibles d'être exercées par ladite orthèse en surface du membre en une pluralité correspondante de points prédéterminés,
dispositif **caractérisé en ce qu'**il comprend en outre :
- des moyens (10) de simulation numérique de l'action de la contention sur l'hémodynamique du retour veineux, aptes à délivrer des valeurs de débit sanguin et/ou de pression intraveineuse en une pluralité de points d'un modèle numérique représentatif du réseau veineux jambier d'un membre, et
- des moyens (26) de présentation desdites valeurs de débit sanguin et/ou de pression intraveineuse déterminées par ces moyens de simulation numérique,
et
- des moyens pour appliquer, en entrée desdits moyens (10) de simulation numérique, lesdites valeurs de pression de contention susceptibles d'être exercées par ladite orthèse en surface du membre en une pluralité correspondante de points prédéterminés,
les moyens de simulation numérique (10) étant également aptes à recevoir en entrée un paramètre de posture (24) parmi la position orthostatique, le décubitus dorsal, la marche et la position assise, et étant aptes à sélectionner ou adapter le modèle numérique du réseau veineux en fonction de ce paramètre de posture,
pour évaluer l'efficacité d'une contention donnée sur le retour veineux, de manière à définir des profils de pression optimisés, puis, à partir des profils optimaux ainsi trouvés, choisir une orthèse dans une gamme préexistante ou fabriquer sur mesure une orthèse d'après les caractéristiques morphologiques d'un patient.

2. Utilisation selon la revendication 1, où lesdits moyens pour produire des valeurs (14) de pression de contention comprennent un ensemble de capteurs de meure de la pression exercée par l'orthèse en surface du membre à l'endroit du capteur.

3. Utilisation selon la revendication 1, où lesdits moyens pour produire des valeurs (16) de pression de contention comprennent :
- des moyens pour établir un premier fichier de données représentatives des caractéristiques morphologiques du membre (20),
- des moyens pour établir un deuxième fichier de données représentatives des caractéristiques dimensionnelles et rhéologiques de l'orthèse (22), et
- des moyens de calcul de cartographie de pression de contention (18), aptes à déterminer lesdites valeurs de pression de contention à partir des données des premier et deuxième fichiers.

4. Utilisation selon la revendication 1, où les moyens (26) de présentation des valeurs de débit sanguin et/ou de pression intraveineuse comprennent des moyens d'affichage d'un graphe orienté valué (30, 36) comprenant une pluralité d'arcs interconnectés (32) représentatifs chacun d'un segment de vaisseau du réseau veineux jambier, ces moyens d'affichage étant aptes à associer à chaque arc du graphe une valeur correspondante de débit sanguin et/ou de pression intraveineuse calculée par les moyens de simulation numérique.

5. Utilisation selon la revendication 4, où les moyens d'affichage sont aptes à appliquer localement à chaque arc (32) du graphe (30, 36) un codage de couleur représentatif de la valeur correspondante de débit sanguin et/ou de pression intraveineuse.

6. Utilisation selon la revendication 4, où les moyens d'affichage comprennent des moyens aptes à produire une représentation plane du graphe (30).

7. Utilisation selon la revendication 4, où les moyens d'affichage comprennent des moyens aptes à produire, en superposition, une représentation tridimensionnelle du graphe (36) et une représentation tridimensionnelle de la surface du membre (38).

## Patentansprüche

1. Verwendung einer Vorrichtung, umfassend:
- Mittel zum Erstellen von Kompressionsdruckwerten, die von der besagten Orthese auf die Oberfläche der Gliedmaße an einer entsprechenden Vielzahl von vorbestimmten Punkten ausgeübt werden können,
wobei die Vorrichtung **dadurch gekennzeichnet ist, dass** sie ferner umfasst:
- Mittel (10) zur digitalen Simulation der Wirkung der Kompression auf die Hämodynamik des venösen Rückflusses, die in der Lage sind, Werte des Blutflusses und/oder des intravenösen Drucks an einer Vielzahl von Punkten eines digitalen Modells bereitzustellen, das für das Beinvenennetz einer Gliedmaße repräsentativ ist, und
- Mittel (26) zum Darstellen der Werte des Blutflusses und/oder des intravenösen Drucks, die durch diese Mittel der digitalen Simulation bestimmt wurden, und
- Mittel zum Anwenden, am Eingang der Mittel (10) zur digitalen Simulation, der besagten Kompressionsdruckwerte, die von der Orthese auf die Oberfläche des Gliedmaße an einer entsprechenden Vielzahl von vorbestimmten Punkten ausgeübt werden können,
wobei die Mittel (10) zur digitalen Simulation gleichermaßen geeignet sind zum Empfangen, beim Eingang, eines Haltungsparameters (24), ausgewählt zwischen der orthostatischen Position, der Rückenlage (Dorsal-Dekubitus), dem Gehen und der Sitzhaltung, und geeignet sind zum Auswählen oder Anpassen des digitalen Modells des Venennetzes als Funktion dieses Haltungsparameters, um die Wirksamkeit einer bestimmten Kompression auf den venösen Rückfluss zu beurteilen und dadurch optimierte Druckprofile zu definieren, wobei sodann basierend auf den so bestimmten optimalen Profilen eine Orthese aus einer vorexistierenden Palette ausgewählt oder eine Orthese maßgeschneidert gemäß den morphologischen Merkmalen eines Patienten angefertigt wird.

2. Verwendung nach Anspruch 1, wobei die Mittel zum Erstellen von Werten (14) des Kompressionsdrucks einen Satz von Sensoren zum Messen des Drucks umfassen, der von der Orthese auf die Oberfläche der Gliedmaße am Ort des Sensors ausgeübt wird.

3. Verwendung nach Anspruch 1, wobei die Mittel zur Erstellung von Kompressionsdruckwerten (16) umfassen:
- Mittel zum Einrichten einer ersten Datei von Daten, die für die morphologischen Merkmale der Gliedmaße repräsentativ sind (20),
- Mittel zum Einrichten einer zweiten Datei von Daten, die für die dimensionalen und rheologischen Merkmale der Orthese repräsentativ sind (22), und
- Mittel zum Berechnen der Kompressionsdruck-Kartographie (18), die geeignet sind, die besagten Kompressionsdruckwerte basierend auf den Daten der ersten und zweiten Datei zu bestimmen.

4. Verwendung nach Anspruch 1, wobei die Mittel (26) zum Darstellen der Werte des Blutflusses und/oder des intravenösen Drucks Mittel zum Anzeigen eines wertorientierten Graphen (30, 36) umfassen, der eine Vielzahl von miteinander verbundenen Bögen (32) umfasst, die jeweils ein Gefäßsegment des Beinvenennetzes repräsentieren, wobei diese Anzeigemittel jedem Bogen des Graphen einen entsprechenden Wert des Blutflusses und/oder des intravenösen Drucks zuordnen können, der durch die Mittel zur digitalen Simulation berechnet wird.

5. Verwendung nach Anspruch 4, wobei die Anzeigemittel in der Lage sind, lokal auf jeden Bogen (32) des Graphen (30, 36) eine Farbcodierung aufzubringen, die für den entsprechenden Wert des Blutflusses und/oder des intravenösen Drucks repräsentativ ist.

6. Verwendung nach Anspruch 4, wobei die Anzeigemittel Mittel umfassen, die in der Lage sind, eine planare Darstellung des Graphen (30) zu erzeugen.

7. Verwendung nach Anspruch 4, wobei die Anzeigemittel Mittel umfassen, die in der Lage sind, in Überlagerung eine dreidimensionale Darstellung des Graphen (36) und eine dreidimensionale Darstellung der Oberfläche der Gliedmaße (38) zu erzeugen.

## Claims

1. Use of a device comprising:
- means for producing compression pressure values capable of being exerted by said orthosis on the surface of the limb at a corresponding plurality of predetermined points,
said device being **characterized in that** it additionally comprises:
- means (10) for numerically simulating the action of the compression on the haemodynamics of the venous return, able to deliver values of blood flow rate and/or intravenous pressure at a plurality of points of a numerical model representative of the sural venous network of a limb, and
- means (26) for presenting said values of blood flow rate and/or intravenous pressure that are determined by these numerical simulation means,
and
- means for applying, as input to said numerical simulation means (10), said compression pressure values capable of being exerted by said orthosis on the surface of the limb at a corresponding plurality of predetermined points,
the numerical simulation means (10) also being able to receive as input a posture parameter (24) from among an orthostatic position, a dorsal decubitus position, a walking position and a seated position, and being able to select or adapt the numerical model of the venous network as a function of this posture parameter,
in order to evaluate the efficacy of a given compression on the venous return, so as to define optimized pressure profiles, then, on the basis of the optimal profiles thus found, to choose an orthosis from a pre-existing range or to manufacture a made-to-measure orthosis according to the morphological characteristics of a patient.

2. Use according to Claim 1, where said means for producing compression pressure values (14) comprise a set of sensors for measuring the pressure exerted by the orthosis on the surface of the limb at the location of the sensor.

3. Use according to Claim 1, where said means for producing compression pressure values (16) comprise:
- means for establishing a first file containing data representative of the morphological characteristics of the limb (20),
- means for establishing a second file containing data representative of the dimensional and rheological characteristics of the orthosis (22), and
- compression pressure mapping calculation means (18) able to determine said compression pressure values on the basis of the data of the first and second files.

4. Use according to Claim 1, where the means (26) for presenting the values of blood flow rate and/or intravenous pressure comprise means for displaying a weighted directed graph (30, 36) comprising a plurality of interconnected arcs (32) each representative of a vessel segment of the sural venous network, these display means being able to associate with each arc of the graph a corresponding value of blood flow rate and/or intravenous pressure calculated by the numerical simulation means.

5. Use according to Claim 4, where the display means are able to apply locally to each arc (32) of the graph (30, 36) a colour coding representative of the corresponding value of blood flow rate and/or intravenous pressure.

6. Use according to Claim 4, where the display means comprise means able to produce a plane representation of the graph (30).

7. Use according to Claim 4, where the display means comprise means able to produce, in overlay, a three-dimensional representation of the graph (36) and a three-dimensional representation of the surface of the limb (38) .
